# EUROPEAN PATENT APPLICATION

(11) **EP 4 846 943 A1**
(43) Date of publication of application: **02.12.2026**
(21) Application number: 25178860.0
(22) Date of filing: 26.05.2025
(51) Int. Cl.: A61B 8/00, A61B 8/08, A61B 8/06

(54) **SYSTEMS AND METHODS FOR VESSEL LUMEN DETECTION AND HEMODYNAMIC MONITORING**

(71) Applicant: Koninklijke Philips N.V., 5656 AE Eindhoven (NL)
(72) Inventor: FRACKOWIAK, Bruno Jean François, 5656 AG Eindhoven (NL); JOSHI, Rohan, 5656 AG Eindhoven (NL); VAN KNIPPENBERG, Luuk Anton Eli Maria, 5656 AG Eindhoven (NL); VERBAKEL, Frank, 5656 AG Eindhoven (NL); SHULEPOV, Sergey Yuryevich, 5656 AG Eindhoven (NL)
(74) Representative: Philips Intellectual Property & Standards

(57) **Abstract**

Provided herein are systems, methods, and computer program products that enable improved tissue boundary detection in ultrasound imaging and thereby improve the accuracy of technologies that require accurate detection of these anatomical structures. **In** accordance with aspects of the present disclosure, one or more B-mode ultrasound images may be processed using two or more directional image processing techniques that account for different orientations of the anatomical structure within the B-mode ultrasound image, which results in improved resolution of the tissue boundaries of the anatomical structure.

## Description

### FIELD OF THE INVENTION

The present disclosure relates generally to medical imaging techniques for detecting tissue boundaries, and finds particular application in systems and methods of detecting vessel lumens and hemodynamic monitoring using ultrasound imaging.

### BACKGROUND OF THE INVENTION

Diagnostics of anatomical structures through ultrasound (US) imaging generally involves one or more types of US imaging, such as structural imaging (e.g., B-mode ultrasound), Doppler imaging (color flow, spectral Doppler), and/or motion imaging (e.g., M-mode ultrasound). Because ultrasound imaging can be used to distinguish between different types of tissues, ultrasound imaging can be used in various applications, such as evaluate different internal organs and/or blood vessels (e.g., veins and arteries).

While ultrasound imaging is generally considered safe and non-invasive for the patient, it often requires a skilled sonographer to set up the ultrasound settings and orient the ultrasound probe manually on the patient skin surface. The traditional way of working in diagnostic ultrasounds is to first apply a standard vendor-optimized preset for a selected study. Using this preset, the ultrasound data acquired on a patient is visualized. Based on this visualization, the sonographer adjusts the universal preset to specifically optimize acquisition for this patient, taking into account specific of the patient's physiology. This is a cumbersome procedure that requires significant trial-and-error to enhance the contrast on the structural imaging and obtain, for example, optimal velocity signals from the Color Doppler or Pulsed Wave Doppler (PWD).

Furthermore, conventional approaches ultrasound imaging of anatomical structures often fails to accurately resolve all portions of the intended anatomical structure due to US wave reflection, refraction, and shadowing effects that distort the imaging results. For example, this can be result in anatomical structures where the "horizontal" tissue boundaries (when viewed in a B-mode image) are well resolved but the "vertical" tissue boundaries (when viewed in a B-mode image) are not. In specific applications, such as hemodynamic monitoring, this results in poor lumen detection and thus inaccurate Doppler measurements.

### SUMMARY OF THE INVENTION

It is an object of the present disclosure to provide systems and methods that improve tissue boundaries detection of anatomical structures, such as organs and blood vessels. In particular applications, the present disclosure provides improved vessel lumen detection for applications such as hemodynamic monitoring, user guidance for ultrasound probe positioning, diagnosis of the vessel wall, and support to stent placement.

According to an embodiment of the present disclosure, a method for detecting tissue boundaries of anatomical structures using an ultrasound imaging probe is provided. The method may include: receiving ultrasound imaging data for an individual, wherein the ultrasound imaging data includes at least a B-mode ultrasound image of an anatomical structure of the individual, the anatomical structure having tissue boundaries that define the anatomical structure; estimating a rough position of the anatomical structure based on at least the B-mode ultrasound image received; determining a full region-of-interest for the anatomical structure based on the rough position of the anatomical structure; determining at least a first subsection of the full region-of-interest and at least a second subsection of the full region-of-interest; and generating an enhanced ultrasound image by applying a first directional image processing technique to at least the first subsection of the full region-of-interest and a second directional image processing technique to at least the second subsection of the full region-of-interest.

In an aspect, the rough position of the anatomical structure may be estimated by approximating a rough centroid of the anatomical structure or by estimating an image intensity range related to fluid within the anatomical structure.

In an aspect, the full region-of-interest may be determined such that the full region-of-interest is centered on the rough centroid approximated for the anatomical structure.

In an aspect, the ultrasound imaging data can further include a Color Doppler data, and the rough position of the anatomical structure can be further estimated based on the Color Doppler data.

In an aspect, the anatomical structure can be a blood vessel lumen.

In an aspect, the first and second directional image processing techniques can be directional image filtering algorithms.

In an aspect, the first directional image processing technique can be a first morphological filtering algorithm having a first kernel, and the second directional image processing technique can be a second morphological filtering algorithm having a second kernel. The first kernel can have a different shape and/or size than the second kernel.

In an aspect, the method can further include: remapping an image intensity profile of the B-mode ultrasound image to generate a processed B-mode ultrasound image; and performing a feature segmentation operation on the enhanced B-mode ultrasound image to identify the tissue boundaries of the anatomical structure of the individual, wherein the enhanced B-mode ultrasound image is generated by applying at least the first and second directional image processing techniques to the processed B-mode ultrasound image.

**In** an aspect, the method can further include: approximating the anatomical structure by fitting an ellipse to a set of points representing the tissue boundaries of the anatomical structure of the individual, wherein the ellipse has a major axis and a minor axis.

According to another embodiment of the present disclosure, a method of monitoring blood flow of an individual is provided. The method can include: detecting the tissue boundaries of an anatomical structure of the individual using an ultrasound imaging probe as described herein, wherein the anatomical structure is a blood vessel lumen; determining probe angles *φ* and *θ* of the ultrasound imaging probe, wherein *φ* corresponds to the orientation of the major axis of the fitted ellipse with respect to a first axis on the B-mode ultrasound image, and wherein *θ* corresponds to the ratio of the minor axis to the major axis of the fitted ellipse; measuring, using the ultrasound imaging probe, Doppler frequency shift data (Δ*f_{d}*) from the blood flow within the blood vessel lumen; and determining a blood flow measurement for the individual based on the Doppler frequency shift data (Δ*f_{d}*) and the probe angles *φ* and *θ*.

According to yet another embodiment of the present disclosure, a system for detecting tissue boundaries of anatomical structures is provided. The system can include: an ultrasound imaging probe configured to generate ultrasound imaging data comprising at least B-mode ultrasound images; a non-transitory computer-readable storage medium having stored thereon computer-readable instructions; and one or more processors in communication with the computer-readable storage medium, wherein the one or more processors are configured by the computer-readable instructions stored thereon to perform the following operations: (i) receive ultrasound imaging data for an individual, wherein the ultrasound imaging data includes at least a B-mode ultrasound image of an anatomical structure of the individual, the anatomical structure having tissue boundaries that define the anatomical structure; (ii) estimate a rough position of the anatomical structure based on at least the B-mode ultrasound image received; (iii) determine a full region-of-interest for the anatomical structure based on the rough position of the anatomical structure; (iv) determine at least a first subsection of the full region-of-interest and at least a second subsection of the full region-of-interest; and (v) generate an enhanced ultrasound image by applying a first directional image processing technique to at least the first subsection of the full region-of-interest and a second directional image processing technique to at least the second subsection of the full region-of-interest.

**In** an aspect, the ultrasound imaging data for the individual can be generated using the ultrasound imaging probe.

In an aspect, the anatomical structure can be a blood vessel lumen.

In an aspect, the first and second directional image processing techniques can be directional image filtering algorithms.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a flowchart of a method for detecting tissue boundaries of anatomical structures using an ultrasound imaging probe in accordance with aspects of the present disclosure.
FIG. 2 is a default B-mode ultrasound image illustrated in accordance with aspects of the present disclosure.
FIG. 3 is a B-mode ultrasound image with a rough centroid annotated in accordance with aspects of the present disclosure.
FIG. 4 is a B-mode ultrasound image with a full region-of-interest annotated in accordance with aspects of the present disclosure.
FIG. 5 is a B-mode ultrasound image with three sub-regions of the full region-of-interest annotated in accordance with aspects of the present disclosure.
FIG. 6 is an illustration showing the application of different imaging processing techniques to different sub-regions of the B-mode ultrasound image in accordance with aspects of the present disclosure.
FIG. 7 is an enhanced B-mode ultrasound image annotated in accordance with aspects of the present disclosure.
FIG. 8 is an illustration of a medical procedure involving ultrasound imaging of an individual's carotid artery in accordance with aspects of the present disclosure.
FIG. 9 is an illustration of an ultrasound imaging beam intersecting with a patient's blood vessel in accordance with aspects of the present disclosure.
FIG. 10 is an illustration of the cross-section of the blood vessel where ultrasound imaging beam passes through in accordance with aspects of the present disclosure.
FIG. 11 is a default B-mode ultrasound image with vessel lumen parameters annotated in accordance with aspects of the present disclosure.
FIG. 12 is a flowchart of a method of monitoring blood flow of an individual in accordance with aspects of the present disclosure.
FIG. 13A is a default B-mode ultrasound image with rough centroid and a centerline annotated in accordance with aspects of the present disclosure.
FIG. 13B is a processed B-mode ultrasound image with the same centerline annotated in accordance with aspects of the present disclosure.
FIG. 14 is a chart illustrating the intensity profile along the centerlines (shown in FIGS. 13A and 13B) in accordance with aspects of the present disclosure.
FIG. 15 is a chart illustrating a non-linear intensity remapping algorithm in accordance with aspects of the present disclosure.
FIG. 16A is a processed B-mode ultrasound image annotated in accordance with aspects of the present disclosure.
FIG. 16B is view of the annotations (shown in FIG. 16A) without the B-mode ultrasound image illustrated in accordance with aspects of the present disclosure.
FIG. 17 is a chart illustrating the intensity profile along a spike (shown in the annotations of FIG. 16B) in accordance with aspects of the present disclosure.
FIG. 18A is a diagram illustrating the determination of a full region-of-interest in accordance with aspects of the present disclosure.
FIG. 18B is a diagram illustrating the determination of multiple subsections of the full region-of-interest in accordance with aspects of the present disclosure.
FIG. 18C is a diagram illustrating an anatomical structure relative to the propagation directions of an ultrasound beam in accordance with aspects of the present disclosure.
FIG. 19A is an enhanced (i.e., post-processing) B-mode ultrasound image annotated in accordance with aspects of the present disclosure.
FIG. 19B is a view of the annotations (shown in FIG. 19A) without the B-mode ultrasound image illustrated in accordance with aspects of the present disclosure.
FIG. 20 is a diagram illustrating a system for detecting tissue boundaries of anatomical structures in accordance with aspects of the present disclosure.
FIG. 21 is a block diagram illustrating various components a system for detecting tissue boundaries of anatomical structures in accordance with aspects of the present disclosure.
FIG. 22 is a graph illustrating the error in a first probe angle in accordance with aspects of the present disclosure.
FIG. 23 is a graph illustrating the error in a second probe angle in accordance with aspects of the present disclosure.

### DETAILED DESCRIPTION OF EMBODIMENTS

Described herein are systems and methods of improved tissue boundary detection in medical imagining, and in particular, ultrasound imaging. Tissue boundary detection, such as organ and vessel lumen detection, serves multiple clinical applications, including but not limited to hemodynamic monitoring, user guidance for ultrasound probe positioning, diagnosis of the vessel wall, and supporting stent placement. However, the tissue boundaries of the imaged anatomical structure may be poorly resolved and therefore not ideal for clinical or diagnostic purposes. For example, assuming B-mode ultrasound imaging where the ultrasound propagation direction is vertical (or within a small steering angle), vessel walls may be well resolved on the upper and lower parts of the anatomical structure but not on the sides due to ultrasound wave reflection, refraction, and shadowing efforts. Accordingly, the systems and methods of the present disclosure improve upon existing medical imaging systems and methods by more accurately identifying tissue boundaries of various anatomical structures.

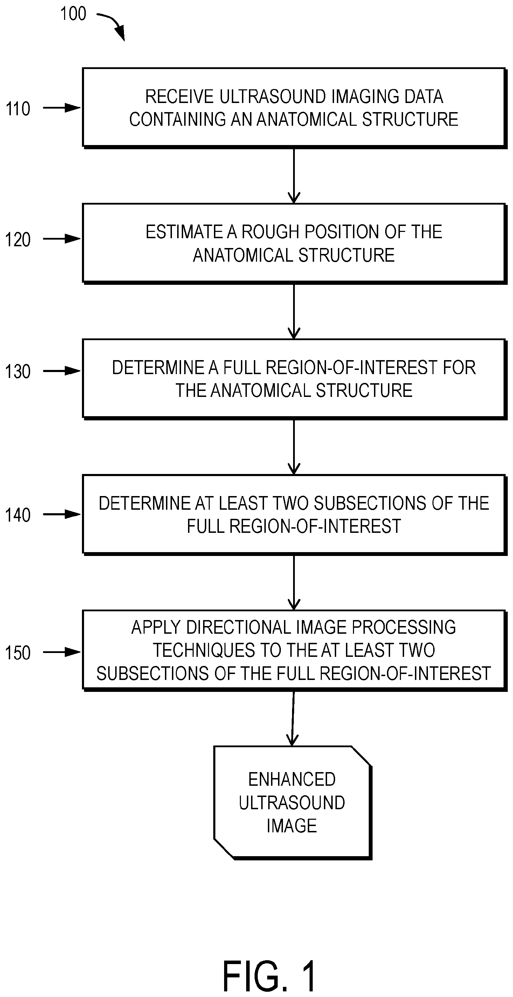

For example, with reference to FIG. 1, a method 100 for detecting tissue boundaries of anatomical structures using an ultrasound imaging probe is provided in accordance with certain aspects of the present disclosure. As shown, the method 100 can include: in a step 110, receiving ultrasound imaging data containing an anatomical structure; in a step 120, estimating a rough position of the anatomical structure; in a step 130, determining a full region-of-interest for the anatomical structure; in a step 140, determining at least two subsections of the full region-of-interest; and in a step 150, applying a different directional image processing techniques to the at least two subsections of the full region-of-interest in order to generate a processed ultrasound image showing the anatomical structure with improved tissue boundary resolution.

More specifically, in the step 110, the method 100 can include receiving ultrasound imaging data for an individual, wherein the ultrasound imaging data includes at least a B-mode ultrasound image of an anatomical structure of the individual. In embodiments, the anatomical structure has tissue boundaries that define the anatomical structure. In particular embodiments, for example, the anatomical structure can be a blood vessel and/or a vessel lumen. As described herein, a lumen refers to the inside space of a tubular structure, including but not limited to an artery, vein, capillary, gastrointestinal tract, bronchi, renal tubules, and/or the like.

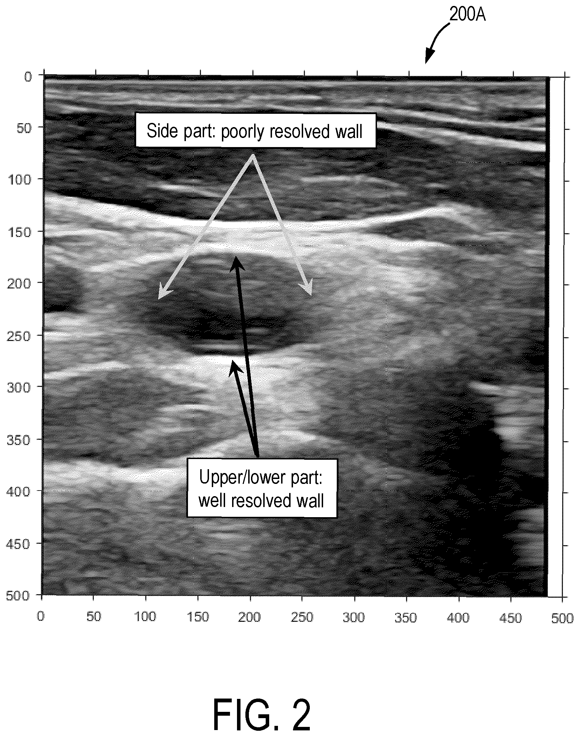

For example, with reference to FIG. 2, the ultrasound imaging data can include one or more B-mode ultrasound images of an individual's carotid artery, similar to the B-mode ultrasound image 200A shown. In the B-mode ultrasound image 200A, it can be seen that the upper and lower wall portions of the artery are well resolved, meaning that the tissue boundaries of the vessel lumen are clear and distinct. In contrast, the side wall portions of the artery are poorly resolved, meaning that the tissue boundaries of the vessel lumen are hazy and indistinct. This can be due to a number of factors, including, but not limited to, ultrasound wave reflection, refraction, and shadowing efforts.

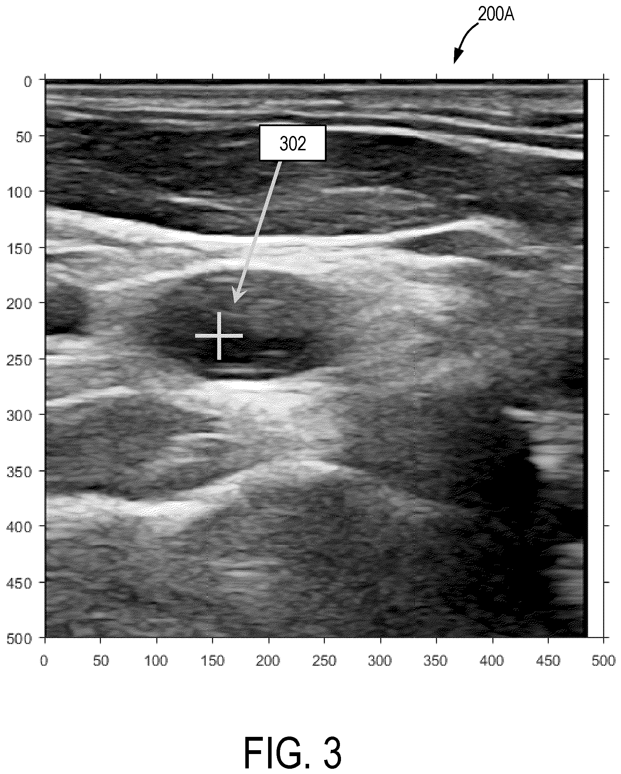

After receiving the ultrasound imaging data including at least one B-mode ultrasound image 200A of an anatomical structure, the method 100 can include, in the step 120, estimating a rough position of the anatomical structure based on at least the B-mode ultrasound image 200A received. For example, with reference to FIG. 3, a rough position (e.g., centroid) 302 is estimated for the carotid artery shown in the B-mode ultrasound image 200A.

In embodiments, the rough position of the anatomical structure is a rough centroid. In particular embodiments, the rough centroid position 302 of the anatomical structure can be obtained from the B-mode ultrasound image 200A through intensity thresholding. For example, in some embodiments, the rough position 302 of the anatomical structure can be determined by estimating an image intensity range related to fluid (e.g., blood, etc.) within / passing through the anatomical structure. In further embodiments, the rough centroid position 302 of the anatomical structure can be obtained from the B-mode ultrasound image 200A in conjunction with color Doppler data. That is, for example, the ultrasound imaging data received in the step 110 may also include Color Doppler data, and the rough position 302 of the anatomical structure may be estimated based on the Color Doppler data.

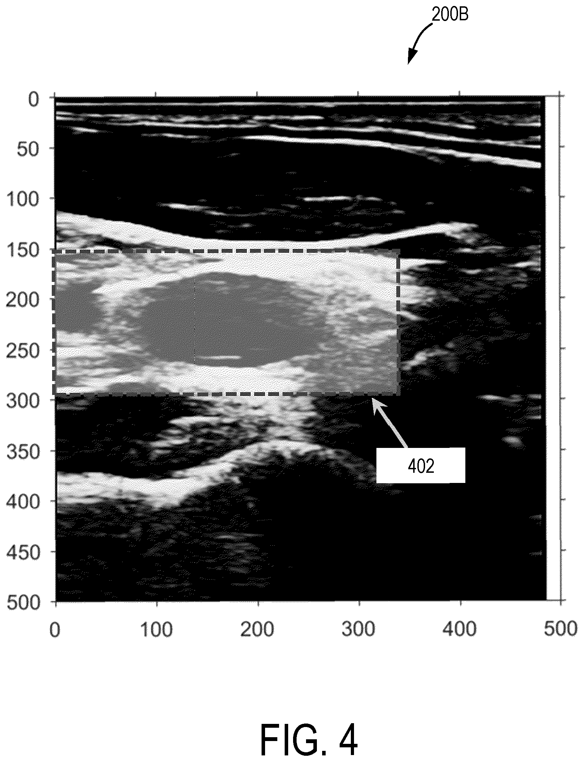

After estimating a rough position 302 of the anatomical structure, the method 100 can include, in a step 130, determining a full region-of-interest for the anatomical structure based on the rough position 302 of the anatomical structure. As described herein, the full region-of-interest corresponds to a subsection of the B-mode image 200A likely to contain the entirety of the desired anatomical structure. For example, with reference to FIG. 4, a full region-of-interest (ROI) 402 is illustrated in connection with a B-mode image 200B of an individual's carotid artery. In embodiments, the full region-of-interest 402 may be centered on the rough centroid 302 approximated for the anatomical structure. Although the full ROI 402 is shown as having a rectangular shape, it should be appreciated that other shapes and geometries can be utilized, including circular, square, or any other type of polygon.

Additionally, as discussed in more detail below, the B-mode ultrasound image 200A may be further processed to obtain the B-mode image 200B. That is, in certain embodiments, the B-mode ultrasound image 200A may be further processed to obtain the B-mode ultrasound image 200B prior to determining the full ROI 402. In other embodiments, the full ROI 402 may be determined before the B-mode ultrasound image 200A is processed to obtain the processed B-mode ultrasound image 200B. That is, the processing may occur before or after the full ROI 402 is defined for the particular B-mode image 200A, 200B. In still further embodiments, it is also contemplated that the B-mode ultrasound image 200A is processed before the rough position 302 of the anatomical structure is estimated.

Then, in a step 140, the method 100 can include determining two or more subsections of the full ROI 402. That is, in embodiments, the step 140 includes splitting up the full ROI 402 into two or more different sub-regions. In particular embodiments, this may include determining at least a first subsection of the full ROI 402 and at least a second subsection of the full ROI 402. In further embodiments, the full ROI 402 may be split up into three or more sub-regions or subsections.

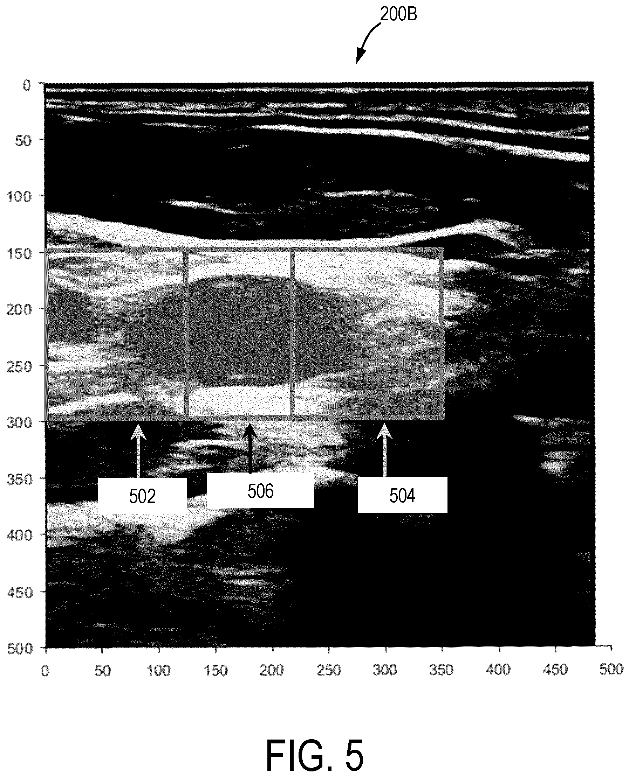

For example, with reference to FIG. 5, the full ROI 402 defined for the anatomical structure shown in the B-mode ultrasound image 200B is divided into three sub-regions or subsections 502, 504, 506. As shown, these sub-regions include two side sub-regions 502, 504 and a single, central sub-region 506. One or more of the sub-regions 502, 504, 506 may contain a portion of the imaged anatomical structure where the tissue boundary is poorly resolved, whereas one or more other sub-regions 502, 504, 506 may contain a portion of the imaged anatomical structure where the tissue boundary is well resolved. In the example of FIG. 5, the side sub-regions 502, 504 each contain portions of the anatomical structure where the tissue boundary is poorly resolved, whereas the central sub-region 506 contains the portions of the anatomical structure where the tissue boundary is well resolved.

In other words, one or more of the sub-regions 502, 504, 506 may divide-up portions of the anatomical structure based on the orientation of the tissue boundary relative to the imaging plane of the imaging modality (e.g., ultrasound imaging). For example, as shown in FIG. 5, the side sub-regions 502, 504 contain portions of the anatomical structure where the tissue boundary is heavily curved and primarily vertical (i.e., roughly aligned with the direction of the ultrasound wave propagation), whereas the central sub-region 506 contains portions of the anatomical structure where the tissue boundary is slight curved and primarily horizontal (i.e., roughly perpendicular to the direction of the ultrasound wave propagation).

Although the sub-regions 502, 504, 506 are shown in FIG. 5 as having a rectangular shape, it should be appreciated that the sub-regions can take on different sizes and geometries. For example, depending on the geometry of the full ROI 402 and/or the anatomical structure being imaged, the number and geometries of the sub-regions may be generated based on predetermined settings or the anticipated shape of the anatomical structure.

After the full ROI 402 is split into two or more sub-regions (e.g., subsections 502, 504, 506), the B-mode ultrasound image 200B may be processed, in the step 150, by applying directional image processing techniques to the sub-regions. In embodiments, two or more directional image processing techniques are applied to the full ROI 402 based on the sub-region determined in the step 140. Thus, in the step 150, the method 100 can include generating a processed ultrasound image showing the anatomical structure with improved tissue boundary resolution by applying a first directional image processing technique to one or more subsections of the full ROI 402, and a second directional image processing to one or more other subsections of the full ROI 402.

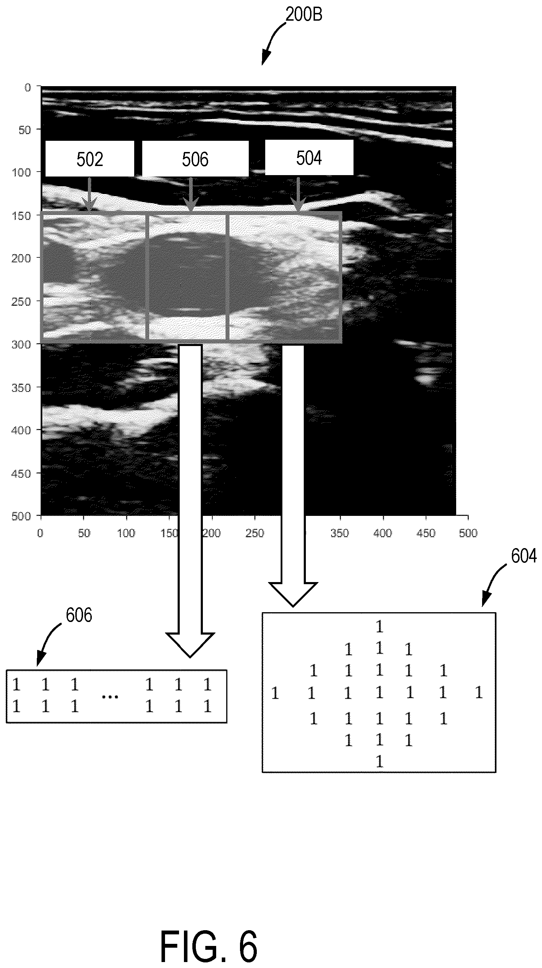

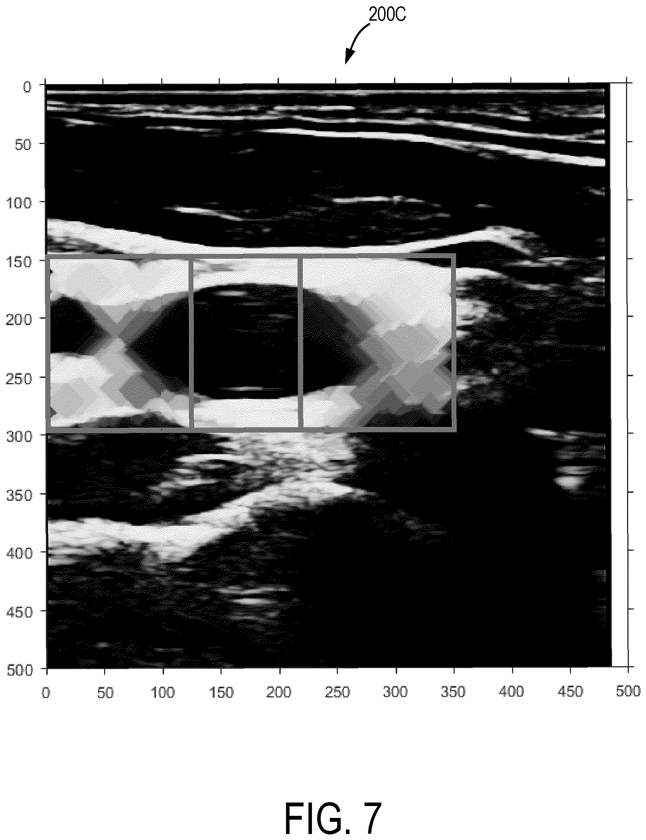

For example, with reference to FIG. 6 and FIG. 7, the B-mode ultrasound image 200B is processed by applying different directional image processing techniques to one or more of the sub-regions 502, 504, 506 to generate the processed B-mode ultrasound image 200C showing the anatomical structure with improved tissue boundary resolution. As shown, a first directional image processing technique is applied to each of the side sub-regions 502, 504 of the full ROI 402, whereas a second directional image processing technique is applied to the central sub-region 506 of the full ROI 402.

In embodiments, one or more of the directional image processing techniques can be directional image filtering algorithms. That is, a first directional image filtering algorithm may be applied to one or more of the sub-regions of the full ROI 402, whereas a different, second directional image filtering algorithm may be applied to one or more other sub-regions of the full ROI 402.

In further embodiments, one or more of the directional image processing techniques can be a morphological filtering algorithm. Thus, in embodiments, a first morphological filtering algorithm having a first kernel may be applied to one or more sub-regions of the full ROI 402, whereas a different, second morphological filtering algorithm having a second kernel may be applied to one or more other sub-regions of the full ROI 402.

In embodiments, the first kernel of the first morphological filtering algorithm can have a different shape and/or size than the second kernel of the second morphological filtering algorithm. For example, as shown in FIG. 6, a first exemplary kernel 604 of a first morphological filtering algorithm is shown as having a diamond shape with a width and height of about seven pixels, and a second exemplary kernel 606 of a second morphological filtering algorithm is shown as having a rectangular shape with a height of about two pixels. In such embodiments, the first kernel 604 may be applied to the side sub-regions 502, 504 of the full ROI 402 while the second kernel 606 may be applied to the central sub-region 506 of the full ROI 402 to generate the processed B-mode ultrasound image 200C showing the anatomical structure with improved tissue boundary resolution.

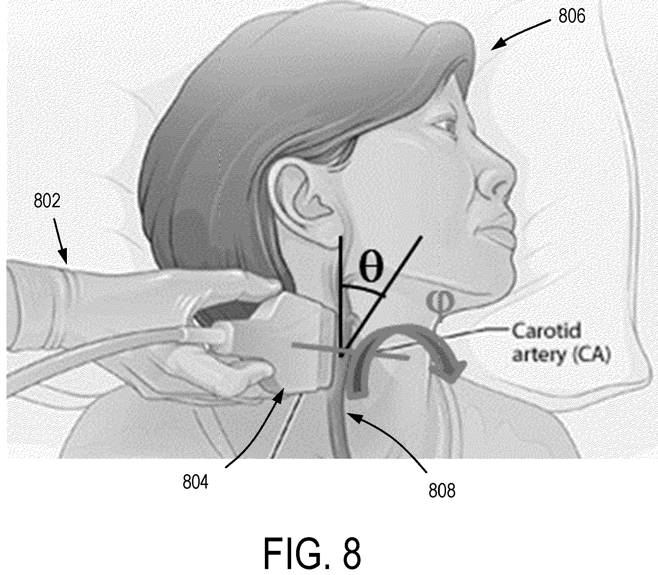

As described above, it is an object of the present disclosure to provide systems and methods that improve vessel lumen detection for applications such as hemodynamic monitoring, user guidance for ultrasound probe positioning, diagnosis of the vessel wall, and support to stent placement. For example, with reference to FIGS. 8-23, the methods described herein (e.g., method 100) are further illustrated in connection with hemodynamic monitoring of an individual. In particular, as shown in the example of FIG. 8, a healthcare professional 802 may utilize an ultrasound probe 804 to measure blood flow for an individual 806 by placing the ultrasound probe 804 against a skin surface of the individual 806 such that a blood vessel 808 such as the carotid artery can be imaged.

Blood flow (e.g., estimated velocity of blood) can be determined from ultrasound Doppler measurements based on the Doppler frequency shift (Δ*f_{d}*) from moving scatterers (e.g., red blood cells). This can be described by the following equation: $v = \frac{c Δ {f}_{d}}{2 cos θ ⋅ sin \left(σ-φ\right) {f}_{t}}$ Eqn. 1 where c is the speed of sound in tissue, *fₜ* is the frequency of the ultrasound wave, *θ* and *φ* are the ultrasound probe angels (as shown in FIG. 8), and *σ* is the steering angle from the ultrasound beam.

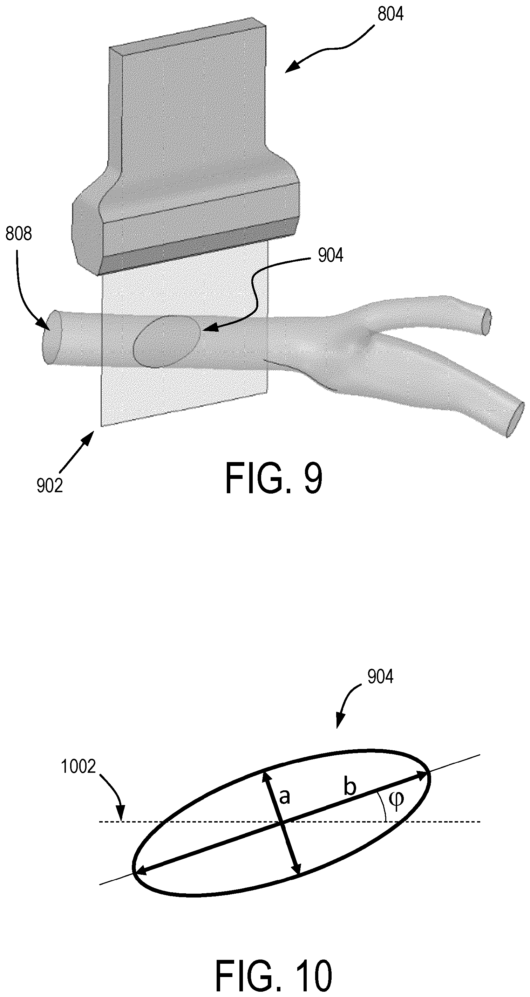

As such, the blood flow waveform estimation from ultrasound Doppler needs accurate knowledge of the ultrasound probe 804 angles and the vessel diameter. The probe angles *θ* and *φ* can be very difficult to control in three-dimensions because the probe 804 is manually positioned on the skin of the individual 806 and the blood vessel orientation with respect to skin surface differs between patients. As shown in FIG. 9 and FIG. 10, when the ultrasound probe 804 has a certain angle with respect to the vessel centerline, the intersection between the blood vessel 808 and the ultrasound probe plane 902 results in an ellipse 904 (assuming a circular shaped vessel, which is the case for the major arteries). By fitting an ellipse 904 on the vessel lumen, probe angles *θ* and *φ* can be determined. In particular, the probe angle *φ* corresponds to the orientation of the major axis with respect to the first axis on the image, while the probe angle *θ* can be determined from the ratio between the major and minor axes of the ellipse according to the following equation: $θ = cos \left(\frac{a}{b}\right)$ Eqn. 2 where *a* is the length of the minor axis of the ellipse and b is the length of the major axis of the ellipse.

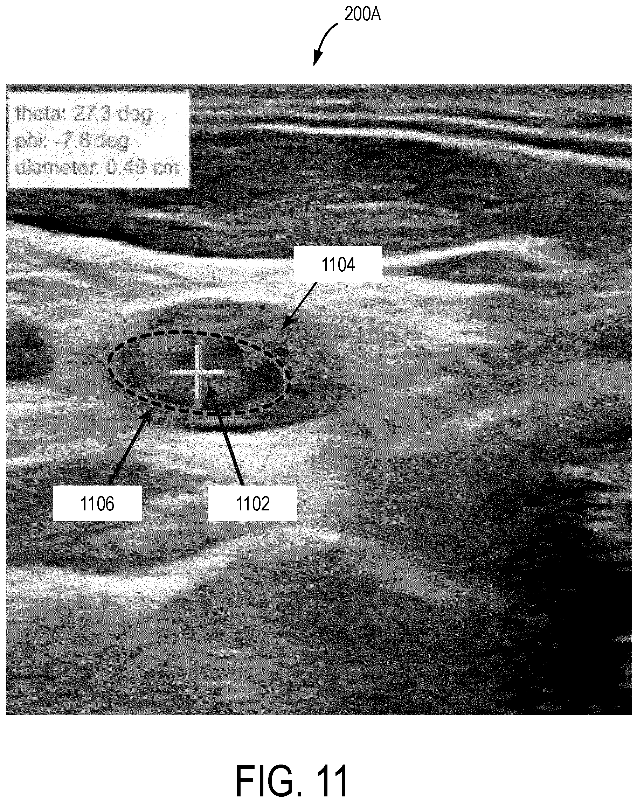

Because these parameters depend on the position and orientation of the vessel lumen 808, it is important that the vessel lumen 808 be accurately detected. For example, with reference to FIG. 11, a rough centroid 1102 for an anatomical structure (i.e., carotid artery) 1104 is estimated based on the intensity profile in the unprocessed B-mode ultrasound image 200A and/or Color Doppler data, and an ellipse 1106 meant to approximate the anatomical structure 1104 is generated. Because the generated ellipse 1106 poorly approximates the vessel lumen 808, 1104, blood flow measurements based on the ellipse 1106 will not be accurate. Thus, in accordance with aspects of the present disclosure, the systems and methods described herein provide for improved tissue boundary detection and therefore improve the accuracy of hemodynamic monitoring.

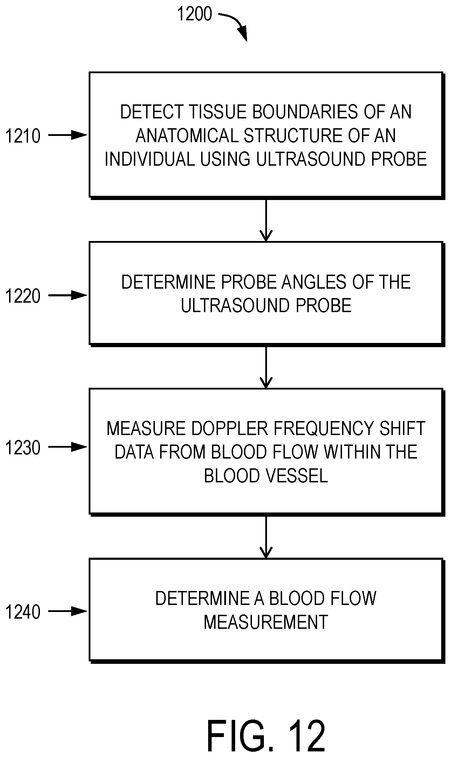

For example, with reference to FIG. 12, a method 1200 for monitoring blood flow of an individual is illustrated in accordance with various aspects of the present disclosure. The method 1200 can include: in a step 1210, detecting the tissue boundaries of an anatomical structure of the individual using an ultrasound imaging probe, wherein the anatomical structure is a blood vessel lumen; in a step 1220, determining probe angles *θ* and *φ* of the ultrasound probe; in a step 1230, measuring Doppler frequency shift data (Δ*f_{d}*) from the blood flow within the blood vessel lumen; and in a step 1240, determining a blood flow measurement for the individual based on the Doppler frequency shift data (Δ*f_{d}*) and the probe angles *φ* and *θ*. In particular embodiments, the blood flow measurement can be an estimate blood flow velocity and the anatomical structure can be the carotid artery of the individual.

More specifically, in the step 1210, the method 1200 can include detecting the tissue boundaries of the anatomical structure of the individual as described with respect to the method 100. That is, the step 1210 can include: receiving ultrasound imaging data for the individual, wherein the ultrasound imaging data includes at least a B-mode ultrasound image of the anatomical structure; estimating a rough position of the anatomical structure based on at least the B-mode ultrasound image received; determining a full region-of-interest for the anatomical structure based on the rough position of the anatomical structure; determining at least a first subsection of the full region-of-interest and at least a second subsection of the full region-of-interest; generating a processed ultrasound image by applying a first directional image processing technique to at least the first subsection of the full region-of-interest and a second directional image processing technique to at least the second subsection of the full region-of-interest.

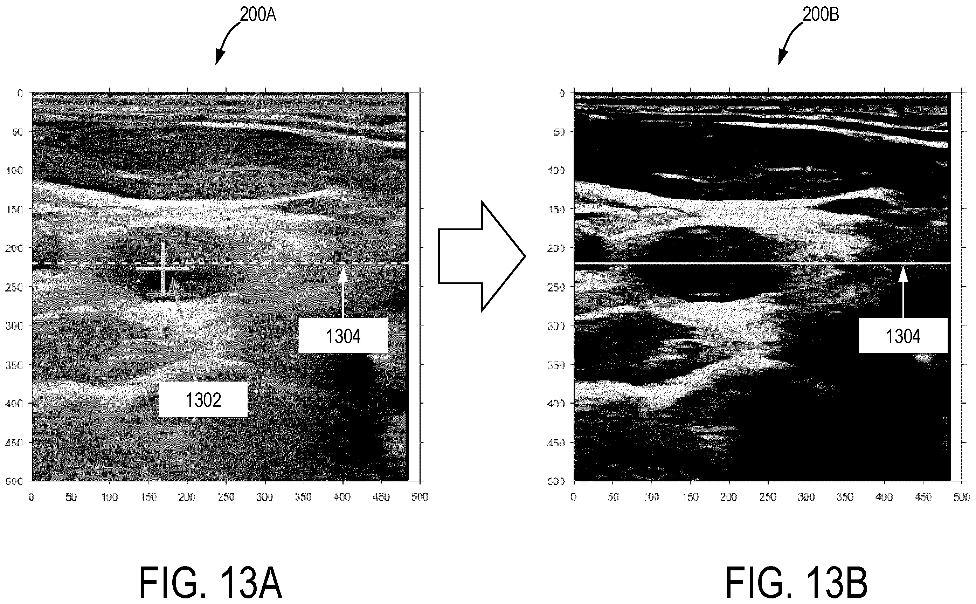

As mentioned above, B-mode ultrasound image(s) received as part of the ultrasound imaging data may be processed before and/or after determining a rough position / centroid of the anatomical structure. For example, with reference to FIG. 13A, a rough centroid 1302 of the anatomical structure is determined using the received B-mode ultrasound image 200A and/or the Color Doppler field (received as part of the ultrasound imaging data).

Then, as shown in the example of FIG. 13B, the received B-mode ultrasound image 200A is processed by remapping the image intensity profile of the B-mode ultrasound image 200A to generate an adjusted B-mode ultrasound image 200B. As described herein, the intensity profile of the B-mode ultrasound image 200A can be remapped to enhance the contour (i.e., tissue boundaries) that are of interest. In embodiments, this remapping operation may be accomplished using histogram equalization, polynomial remapping, and/or other non-linear remapping functions of the ultrasound signal into pixels grey values. As a result of the remapping, the signal to noise ratio (SNR), contrast to noise ratio (CNR), and the contrast gradient at the tissue boundaries (i.e. lumen walls) is improved.

In particular embodiments, the intensity profile of the B-mode ultrasound image 200A is remapped to generate the adjusted B-mode ultrasound image 200B using a non-linear remapping function defined in the logarithmic range, where the main driving parameter is a cut off value *dB_{cut}* and where additional parameters *I_{processed_maxed}* (related to the maximal intensity scale) and *dB_{sigma}* (related to the stretch of the intensity scale) are used to tune the remapping according to the following equations: $dB = 20 ⋅ {log}_{10} \left[\frac{I}{{I}_{max}}\right]$ Eqn. 3 $dB \leq - {dB}_{cut} ⇒ {dB}_{processed} = - {dB}_{cut}$ Eqn. 4 ${I}_{processed} = \frac{{I}_{process _ max}}{1 + {\left({I}_{process _ max}\right)}^{- \frac{\left|{dB}_{processed}+{dB}_{cut}\right|}{{dB}_{sigma}}}}$ Eqn. 5

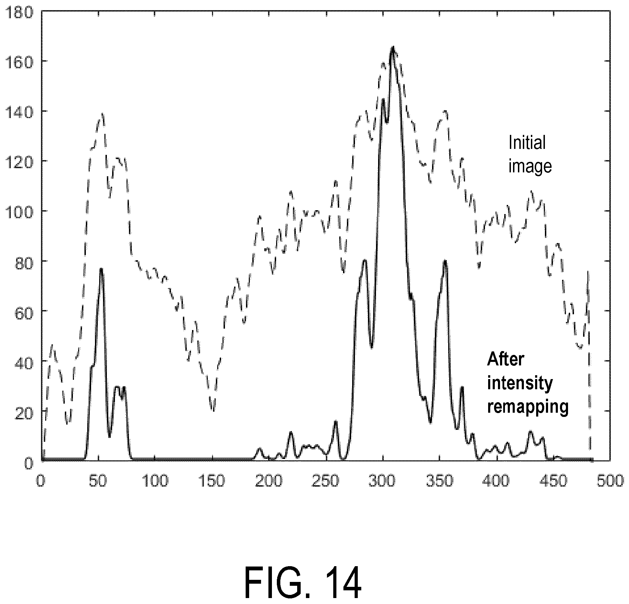

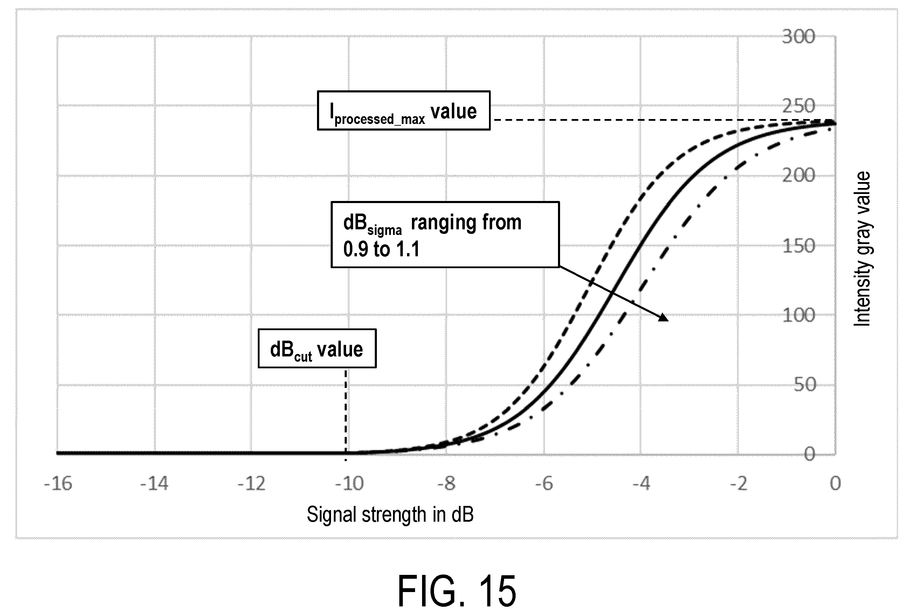

In embodiments, the tuning parameters *dB_{cut}*, *I_{processed_max}*, and *dB_{sigma}* can be adjusted from the image pixel values to obtain an optimal adjusted B-mode ultrasound image 200B. As shown in FIG. 14, for example, the intensity values across profile line 1304 is graphed out moving from the left side of the images 200A, 200B to the right side of the images 200A, 200B. The non-linear remapping function using in the examples of FIG. 13B and FIG. 14 is also illustrated in the graph shown in FIG. 15.

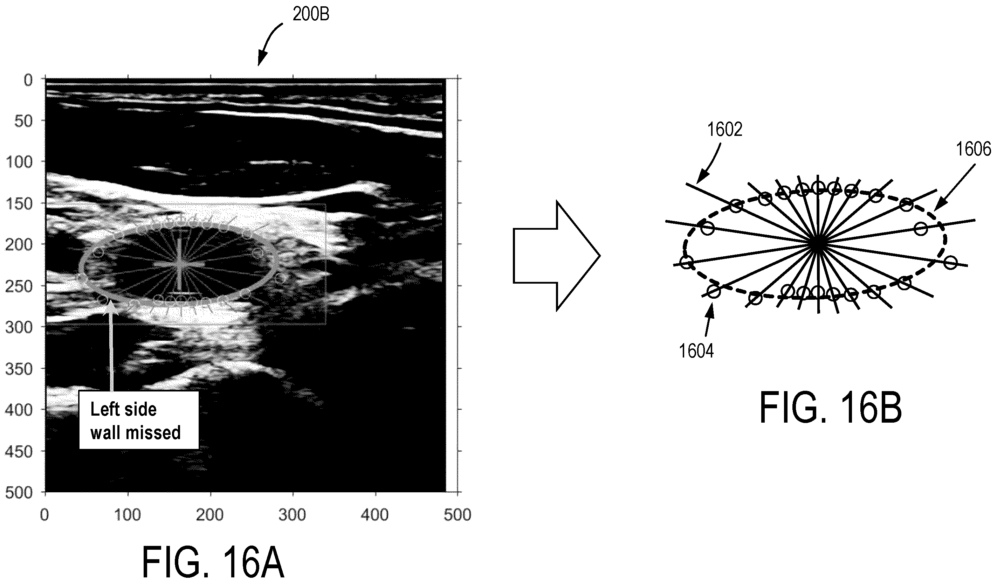

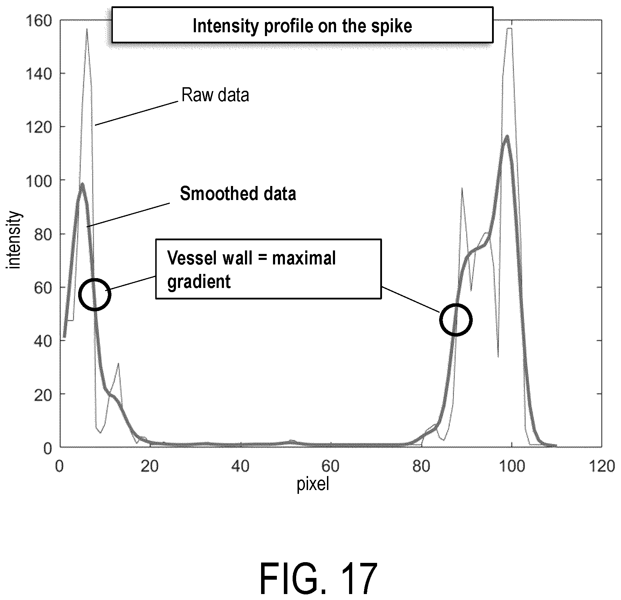

Once the adjusted B-mode ultrasound image 200B is generated, a full region-of-interest (e.g., full ROI 402) can be generated as described above. In particular embodiments, the process of determining a full region-of-interest for the anatomical structure can include fitting a rough outline (e.g., ellipse) to the anatomical structure based on the adjusted B-mode ultrasound image 200B. In particular, with reference to FIG. 16A and 16B, the vessel lumen (e.g., lumen 1104) is roughly detected on the remapped B-mode image 200B by growing spikes 1602 in multiple directions using the rough detected centroid. In embodiments, the detection of the vessel wall on those spikes (indicated by the circles 1604) can rely on Sobel filtering to smooth the intensity profile combined with maximal gradient detection, as shown in FIG. 17. This step results in a set of points representing the tissue boundaries (e.g., blood vessel wall around the lumen). A rough ellipse 1606 can then be fitted on those tissue boundaries points as shown in FIG. 16B, which can subsequently be used to determine a full region-of-interest for the anatomical structure as shown in FIG. 17.

In particular embodiments, it is contemplated that potential outlier points in the set of points representing the tissue boundaries may be filtered and removed, for example, by using a Hampel filter on the distance between each point and the rough centroid. Alternative methods for detecting the anatomical structure are also considered, such as spline or polynomial fitting on the intersecting points between the spikes and the tissue boundaries.

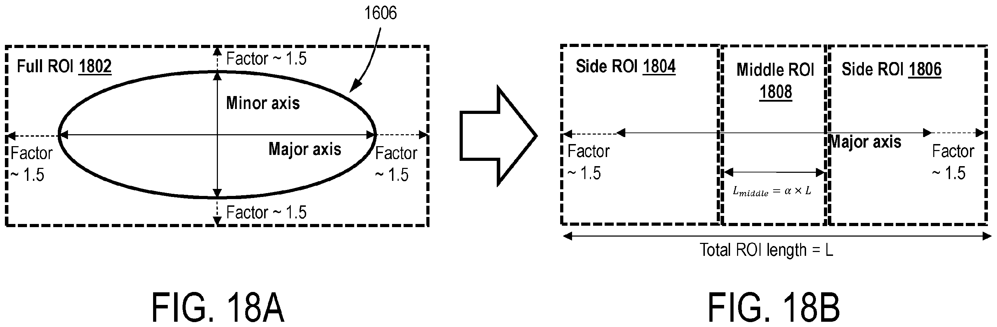

Once the anatomical structure is roughly identified, a full region-of-interest (ROI) 1802 may be generated as shown in FIG. 18A and 18B. In embodiments, the full ROI 1802 can be a predetermined geometry (e.g., rectangular, etc.) with dimensions based on the major and minor axes of the rough-fitted ellipse 1606. In embodiments, the full ROI 1802 can be defined as a rectangle whose length and width are estimated respectively from the major and minor axes, with an enlargement factor. In embodiments, the enlargement factor should be minimized in order to ensure fast computational speed. Thus, in particular embodiments, the enlargement factor is greater than 1.0, including about 1.1, 1.5, 2.0, and/or 2.5.

After determining a full ROI 1802 for the anatomical structure, the next step (as also described above), it to determine two or more subsections or sub-regions of the full ROI 1802. As shown in FIG. 18B, for example, the full ROI 1802 may be split into three sub-regions 1804, 1806, 1808, including a central ROI 1808 covering the upper and lower parts of the anatomical structure and two side ROIs 1804, 1806 covering each side of the anatomical structure.

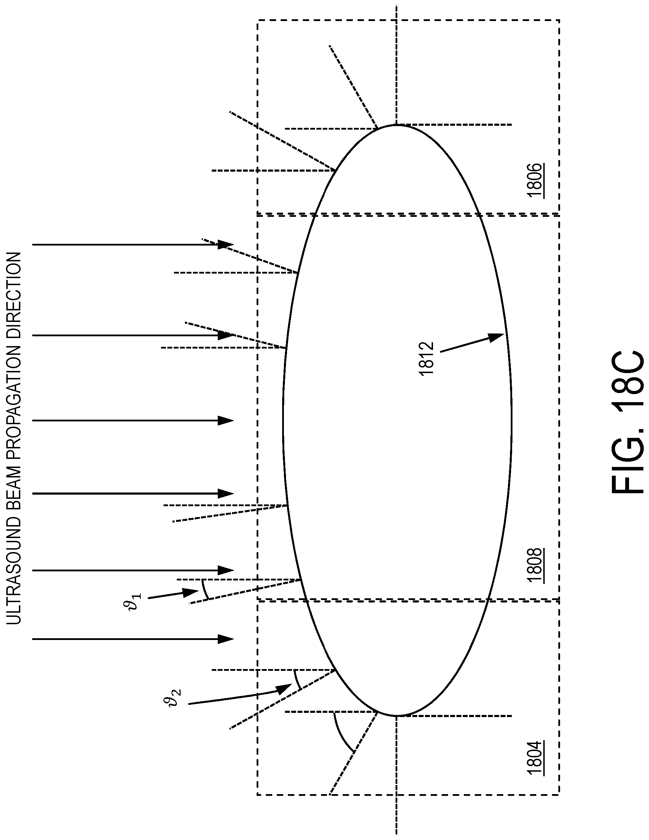

In some embodiments, the different subsections of the full ROI 1802 may be determined based the angle between the normal of the edge and the ultrasound beam propagation direction. For example, as shown in FIG. 18C, the doppler angle theta (e.g., ϑ₁, ϑ₂, etc.) between the normal of the edge of the anatomical structure 1812 and the ultrasound beam propagation direction changes across the imaging plane. Where there is a relatively small doppler angle theta, at least a first subregion (e.g., subregion 1808) may be defined, and where there is a relatively large doppler angle theta, at least a second subregion (e.g., subregions 1804, 1806) may be defined.

In particular embodiments, the size of the central ROI 1808 (*L_{middle}*) can be determined based on the length of the full ROI 1802 rectangle according to the following equation: ${L}_{middle} = α ⋅ L$ Eqn. 6 where a typical *α* value is approximately 0.27. However, it should be appreciated that the *α* value may change depending on the size / shape of the anatomical structure being imaged. That is, in embodiments, the fitted shape may not be an ellipse and may depend on the size /shape of the anatomical structure being imaged.

Then, two or more different directional image processing techniques are applied to the subsections 1804, 1806, 1808 of the full ROI 1802 to generate an enhanced B-mode ultrasound image 200C. For example, as described above with respect to FIG. 6 and FIG. 7, the B-mode ultrasound image 200B can processed by applying different directional image processing techniques to one or more of the sub-regions 1804, 1806, 1808 to generate the processed B-mode ultrasound image 200C showing the anatomical structure with improved tissue boundary resolution.

In embodiments, one or more of the directional image processing techniques are directional image filtering algorithms. That is, a first directional image filtering algorithm may be applied to one or more of the sub-regions of the full ROI 1802, whereas a different, second directional image filtering algorithm may be applied to one or more other sub-regions of the full ROI 1802. In further embodiments, one or more of the directional image processing techniques can be a morphological filtering algorithm. Thus, in embodiments, a first morphological filtering algorithm having a first kernel may be applied to one or more sub-regions of the full ROI 1802, whereas a different, second morphological filtering algorithm having a second kernel may be applied to one or more other sub-regions of the full ROI 1802.

As described herein, the different kernels (e.g., kernels 604, 606) can have different sizes and/or geometries. For example, as shown in FIG. 6, the kernel 606 for the sub-region 506 covering the upper and lower portions of the lumen may have a rectangular shape to enhance the filtering in the horizontal direction (i.e., roughly perpendicular to the ultrasound beam propagation direction). In contrast, the kernels (e.g., kernel 604) for the sub-regions 502, 504 covering each side of the lumen may have a diamond shape to enhance the filtering in a diagonal direction (e.g., a 45° angle). However, it should be appreciated that other sizes and/or geometries can also be used, including but not limited to, circular, square, oval, and/or other polygons.

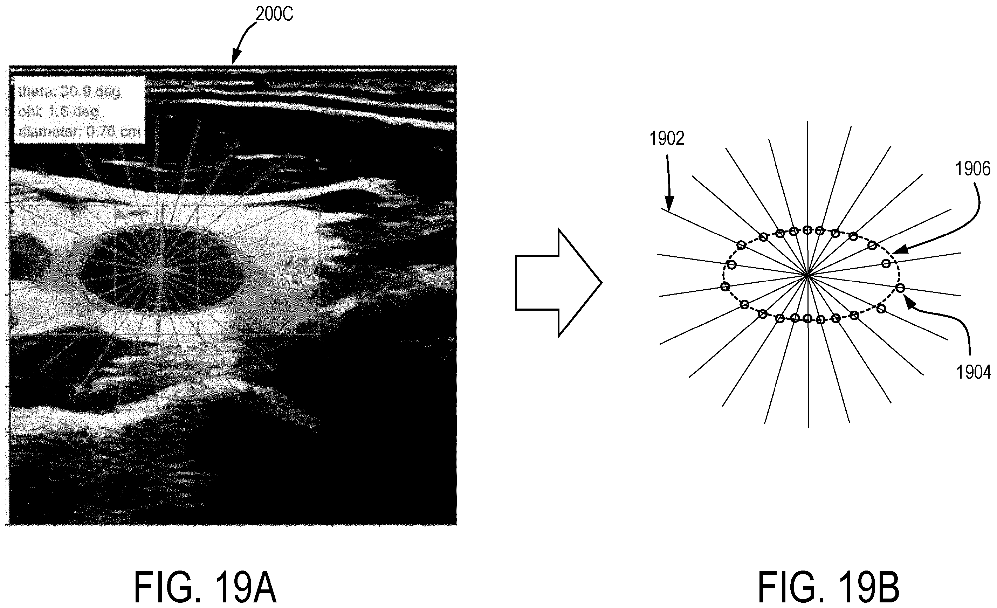

With further reference to FIG. 12, the step 1210 of the method 1200 can then include performing a feature segmentation operation on the enhanced B-mode ultrasound image 200C to identify the tissue boundaries of the anatomical structure of the individual. For example, as shown in FIG. 19A and FIG. 19B, the anatomical structure may be more accurately identified from the enhanced B-mode ultrasound image 200C using a similar method as described above, whereby spikes 1902 are grown in multiple directions using the rough detected centroid. The detection of the tissue boundaries on those spikes (indicated by circles 1904) can similarly rely on Sobel filtering to smooth the intensity profile combined with maximal gradient detection. This step results in a second set of points representing the tissue boundaries (e.g., blood vessel wall around the lumen) that is more accurate than before. A new ellipse 1906 can then be fitted onto those tissue boundary points 1904. It is contemplated that potential outlier points in the second set of points representing the tissue boundaries may be filtered and removed before fitting the ellipse 1906, for example, by using a Hampel filter on the distance between each point and the rough centroid. Alternative methods for detecting the anatomical structure are also considered, such as spline or polynomial fitting on the intersecting points between the spikes and the tissue boundaries.

Next, in the step 1220, the method 1200 can include determining probe angles *φ* and *θ* of the ultrasound imaging probe (e.g., probe 804). In embodiments, *φ* corresponds to the orientation of the major axis (e.g., major axis 'b' shown in FIG. 10) of the fitted ellipse (e.g., ellipse 1906) with respect to a first axis (e.g., axis1002 shown in FIG. 10) on the B-mode ultrasound image (e.g., image 200C). Thus, the probe angle *φ* cam be determined by comparing the major axis of the fitted ellipse with a first axis 1002 on the B-mode ultrasound image. In further embodiments, the probe angle *θ* corresponds to the ratio of the minor axis (e.g., minor axis 'a' shown in FIG. 10) to the major axis (e.g., major axis 'b' shown in FIG. 10) of the fitted ellipse (e.g., ellipse 1906 shown in FIG. 19B). In particular, the probe angle *θ* can be determined according to Equation 2 (discussed above).

In a step 1230, the method 1200 can include using the ultrasound probe (e.g., probe 804) to measure Doppler imaging data representative of a fluid flow within the anatomical structure. In embodiments, for example, the anatomical structure is a blood vessel lumen and the fluid flow is blood flow. In some embodiments, the Doppler imaging data comprises Doppler frequency shift data (Δ*f_{d}*)*.* That is, the moving red blood cells of the blood flow scatter the frequencies of the ultrasound waves emitted from the probe (resulting in a Doppler frequency shift) and the returning signal echoes are then measured by the probe such that the Doppler-frequency shift can be determined.

The method 1200 can then include, in a step 1240, determining a fluid flow measurement for the individual based on the Doppler frequency shift data (Δ*f_{d}*) and the probe angles *φ* and *θ*. In particular embodiments, the anatomical structure can be a blood vessel lumen and the fluid flow can be blood flow. In such embodiments, the fluid flow measurement can be a blood flow parameter such as estimated velocity (v) of the blood flow through the blood vessel lumen. In specific embodiments, the estimated velocity (v) of the blood flow according to Equation 1 (described above) based on the probe angles *φ* and *θ* determined using the image processing techniques described herein.

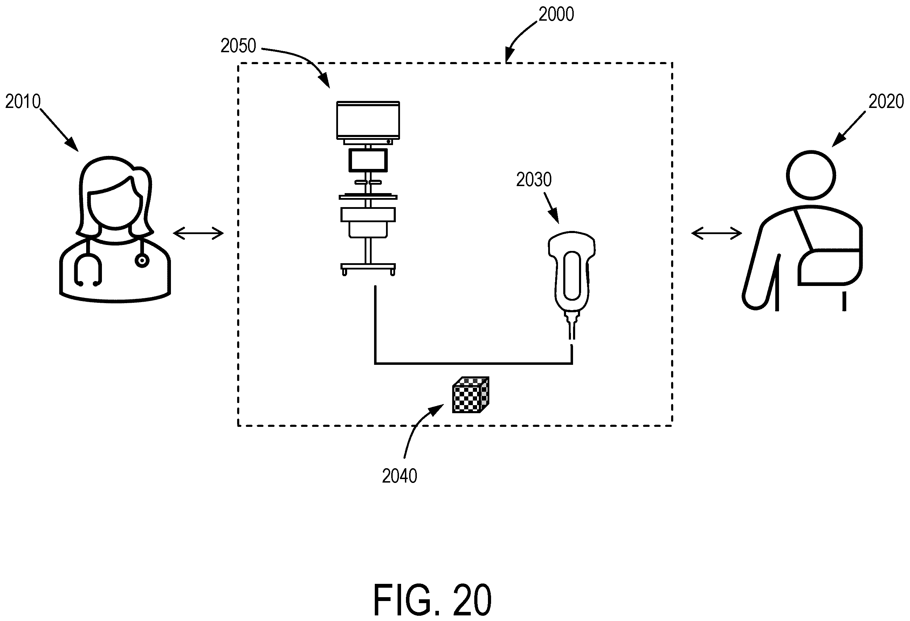

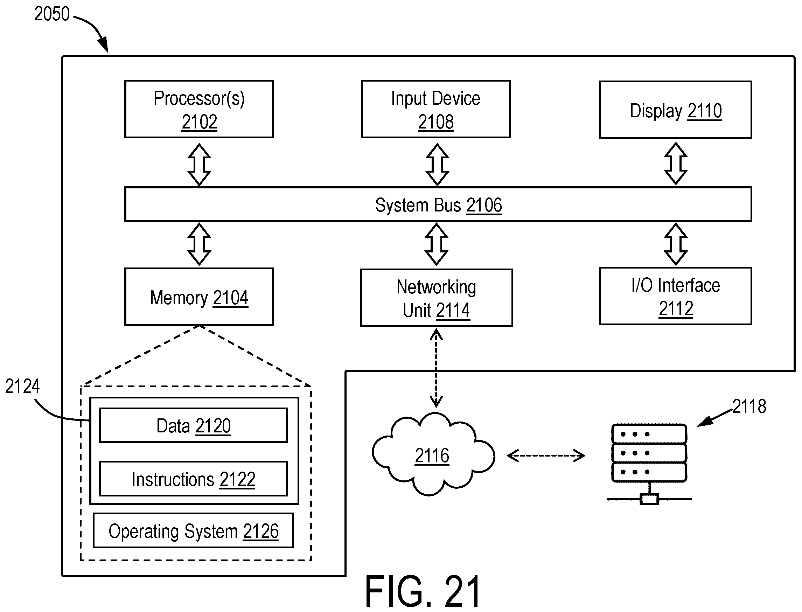

Turning to FIG. 20 and FIG. 21, also provided herein are systems 2000 adapted to detect tissue boundaries of anatomical structures. In embodiments, the systems 2000 can also be configured to assist in various applications that depend on accurately detecting tissue boundaries of anatomical structures, including but not limited to, hemodynamic monitoring, user guidance for ultrasound probe position, diagnosis of vessel wall abnormalities, user support for stent placement, and/or the like. That is, the system 2000 may be used by a healthcare professional 2010 in order to detect the tissue boundaries of an anatomical structure in an individual 2020, and thereby assist in hemodynamic monitoring of the individual 2020, provide ultrasound probe placement guidance to the professional 2010, assist in the diagnosis of vessel wall abnormalities, provide support for stent placement, and/or the other similar applications. As shown in FIG. 20, the system 2000 can include: an ultrasound probe 2030 configured to generate ultrasound imaging data 2040 including one or more B-mode ultrasound images and/or Doppler imaging data; and a monitoring device 2050 comprising a non-transitory computer-readable medium and one or more processors configured to perform one or more operations of the methods (e.g., method 100, 1200) described herein.

As shown in the example of FIG. 21, the monitoring device 2050 can include one or more processors 2102 and a computer-readable memory 2104 interconnected and/or in communication via a system bus 2106 containing conductive circuit pathways through which instructions (e.g., machine-readable signals) may travel to effectuate communication, tasks, storage, and the like. The monitoring device 2050 can be connected to a power source (not shown), which can include an internal power supply and/or an external power supply. In embodiments, the monitoring device 2050 can also include one or more additional components, such as a display 2110, an input device 2108, an input/output (I/O) interface 2112, a networking unit 2114, and the like, including combinations thereof. As shown, each of these components may be interconnected and/or in communication via the system bus 2106, for example.

In embodiments, the one or more processors 2102 can include one or more high-speed data processors adequate to execute the program components described herein and/or perform one or more operations of the methods described herein. The one or more processors 2102 may include a microprocessor, a multi-core processor, a multithreaded processor, an ultra-low voltage processor, an embedded processor, and/or the like, including combinations thereof. The one or more processors 2102 can include multiple processor cores on a single die and/or may be a part of a system on a chip (SoC) in which the processor 2102 and other components are formed into a single integrated circuit, or a single package. That is, the one or more processors 2102 may be a single processor, multiple independent processors, or multiple processor cores on a single die.

In embodiments, the display device 2110 may be configured to display information, including text, graphs, and/or the like. In particular embodiments, the display device 2110 may be configured to a graphical user interface comprising the ultrasound imaging data 2040 and the outputs of the methods described herein. The display device 2110 can include, but is not limited to, a liquid crystal display (LCD), a light-emitting diode (LED) display, a touch screen or other touch-enabled display, a foldable display, a projection display, and so on, or combinations thereof.

In embodiments, the input device 2110 may be configured to receive various forms of input from a user associated with the monitoring device 2050. The input device 2108 can include, but is not limited to, one or more of a keyboard, keypad, trackpad, trackball(s), capacitive keyboard, controller (e.g., a gaming controller), computer mouse, computer stylus / pen, a voice input device, and/or the like, including combinations thereof.

In embodiments, the input/output (I/O) interface 2112 may be configured to connect and/or enable communication with one or more peripheral devices (not shown), including but not limited to additional machine-readable memory devices, diagnostic equipment, and other attachable devices. The I/O interface 2112 may include one or more I/O ports that provide a physical connection to the one or more peripheral devices. In some embodiments, the I/O interface 2112 may include one or more serial ports.

In embodiments, the networking unit 2114 may include one or more types of networking interfaces that facilitate wired and/or wireless communication between the monitoring device 2050 and one or more external devices. That is, the networking unit 2114 may operatively connect the monitoring device 2050 to one or more types of communications networks 2116, which can include a direction interconnection, the Internet, a local area network ("LAN"), a metropolitan area network ("MAN"), a wide area network ("WAN"), a wired or Ethernet connection, a wireless connection, a cellular network, and similar types of communications networks, including combinations thereof. In some embodiments, the monitoring device 2050 may communicate with one or more remote / cloud-based servers and/or cloud-based services, such as remote server 2118, via the communications network 2116.

In embodiments, the memory 2104 can be variously embodied in one or more forms of machine accessible and machine-readable memory. In some embodiments, the memory 2104 includes a storage device (not shown), which can include, but is not limited to, a non-transitory storage medium, a magnetic disk storage, an optical disk storage, an array of storage devices, a solid-state memory device, and/or the like, as well as combinations thereof. The memory 2104 may also include one or more other types of memory, such as dynamic random-access memory (DRAM), static random-access memory (SRAM), erasable programmable read-only memory (EPROM), electrically erasable programmable read-only memory (EEPROM), Flash memory, and/or the like, as well as combinations thereof. In embodiments, the memory 2104 may include one or more types of transitory and/or non-transitory memory.

The monitoring device 2050 can be configured by software components forming an image processing package 2124 that is stored in the memory 2104. The monitoring device 2050 can be configured by the image processing package 2124 to perform one or more processes of the methods described herein. More specifically, the memory 2104 can be configured to store data / information 2120 and computer-readable instructions 2122 that, when executed by the one or more processors 2102, causes the monitoring device 2050 to perform at least the following operations: (i) receive ultrasound imaging data 2040 for an individual 2020, wherein the ultrasound imaging data 2040 includes at least a B-mode ultrasound image of an anatomical structure of the individual 2020, the anatomical structure having tissue boundaries that define the anatomical structure; (ii) estimate a rough position of the anatomical structure based on at least the B-mode ultrasound image received; (iii) determine a full region-of-interest for the anatomical structure based on the rough position of the anatomical structure; (iv) determine at least a first subsection of the full region-of-interest and at least a second subsection of the full region-of-interest; and (v) generate a processed ultrasound image by applying a first directional image processing technique to at least the first subsection of the full region-of-interest and a second directional image processing technique to at least the second subsection of the full region-of-interest.

In further embodiments, the image processing package 2124 can further include computer-readable instructions 2122 that, when executed by the one or more processors 2102, causes the monitoring device 2050 to perform at least the following operations: detecting the tissue boundaries of an anatomical structure of the individual 2020 using an ultrasound probe 2030 as described above; determining probe angles *φ* and *θ* of the ultrasound imaging probe 2030, wherein *φ* corresponds to the orientation of the major axis of a fitted ellipse with respect to a first direction on the B-mode ultrasound image, and wherein *θ* corresponds to the ratio of the minor axis to the major axis of the fitted ellipse; measuring, using the ultrasound imaging probe 2030, Doppler frequency shift data (Δ*f_{d}*) from a fluid flow through the anatomical structure; and determining a fluid flow measurement for the individual based on the Doppler frequency shift data (Δ*f_{d}*) and the probe angles *φ* and *θ*.

In embodiments, the monitoring device 2050 may also include an operating system component 2126, which may be stored in the memory 2104. The operating system component 2126 may be an executable program facilitating the operation of the monitoring device 2050 and/or the ultrasound probe 2030. Typically, the operating system component 2126 can facilitate access of the I/O interface 2112, network interface 2114, the input device 2108, and the display 2110, and can communicate or control other components of the monitoring device 2050.

In accordance with further aspects of the present disclosure, also provided herein is a computer program product 2124 comprising a non-transitory computer-readable storage medium 2104 having stored thereon computer-readable instructions 2122 that, when executed by one or more processors (such as processors 2102), cause the one or more processors to perform one or more operations of the methods described herein (e.g., methods 100, 1200).

As provided herein, the systems, methods, and computer program products enable improved tissue boundary detection in ultrasound imaging, and thereby improve upon technologies requiring accurate detection of anatomical structures. In accordance with certain aspects of the present disclosure, it is contemplated that the systems, methods, and computer program products described herein may include or be used in conjunction with a wearable ultrasound sensor for continuous or semi-continuous hemodynamic monitoring, which would require continuous sensor orientation adjustments due to patient motion (e.g., breathing, yawning, coughing, talking, etc.). Put another way, in applications where continuous or semi-continuous hemodynamic monitoring is desired, the systems, methods, and computer program products of the present disclosure enable consistent, real-time adjustments of sensor orientation that account for unpredictable patient monitions due to breathing, yawning, coughing, talking, and/or the like.

In some embodiments, the systems, methods, and computer program products described herein may can be used to: (1) accurately assess vessel shape and position in order to place a pulsed wave Doppler gate for hemodynamic monitoring; (2) determine ultrasound probe angles *φ* and *θ* for real-time guidance of probe positioning; (3) support detection of plaques, calcifications, or vessel stenosis; and (4) provide guidance to help a healthcare professional position a stent in the artery, among other uses.

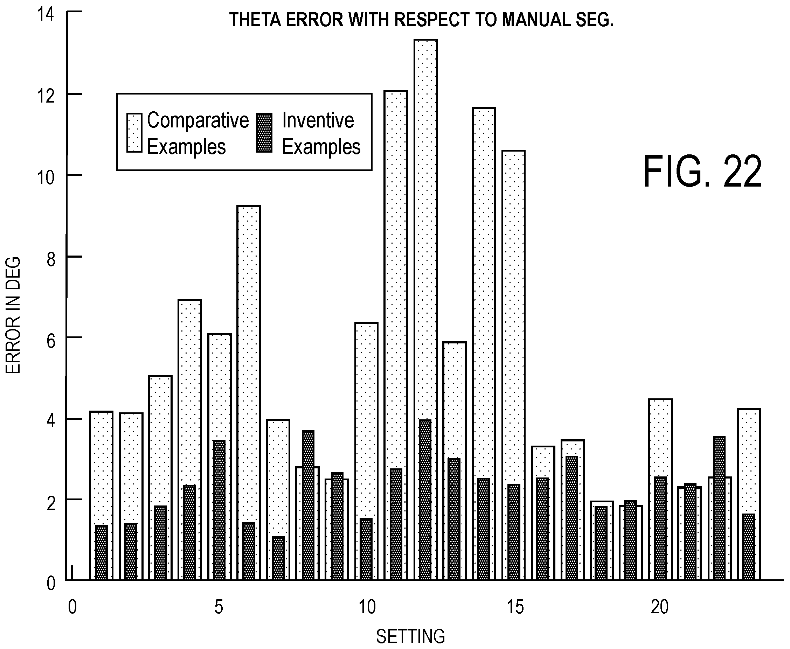

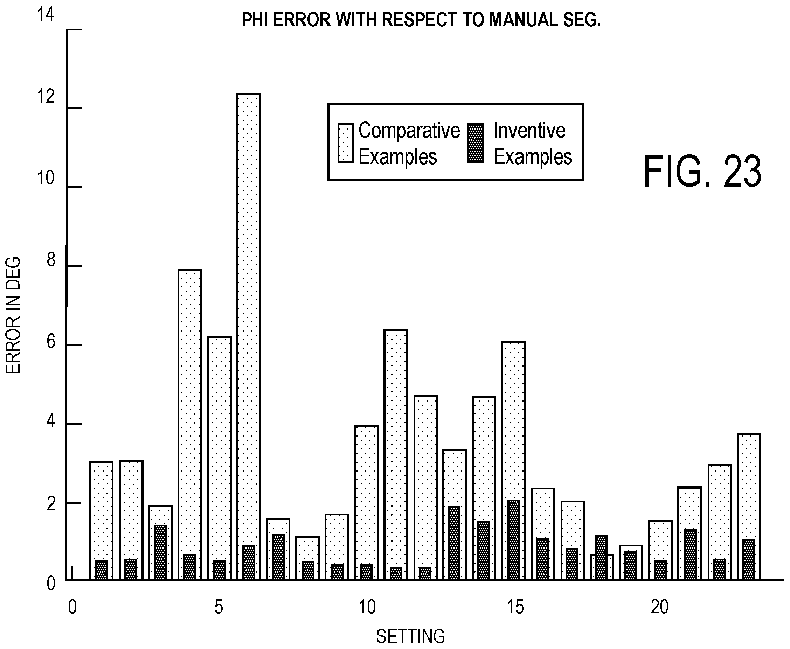

With reference to FIG. 22 and FIG. 23, the systems, methods, and computer program products described herein exhibit significant improvements in the calculation of probe angles over a range of settings when compared with conventional approaches. For example, compared to an ellipse fitting on Color Doppler images (i.e., the comparative method), the angles *φ* and *θ* resulting from the present disclosure are more stable and closer to a manually segmented lumen ground truth (lower error on the bar charts shown in the figures. Specifically, on average over the whole range of settings, the error is significantly reduced using the systems, methods, and computer program products of the present disclosure. These results are also summarized in the tables below:

**Table 1. Statistics on θ Angle Over Whole Set of Settings**

| Statistics on ***θ*** Angle | Average Error | Standard Deviation |
|---|---|---|
| From Comparative Example | 5.61° | 3.47° |
| From Inventive Example | 2.42° | 0.88° |

**Table 2. Statistics on φ Angle Over Whole Set of Settings**

| Statistics on ***φ*** Angle | Average Error | Standard Deviation |
|---|---|---|
| From Comparative Example | 9.17° | 6.77° |
| From Inventive Example | 2.03° | 1.07° |

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. **In** the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for detecting tissue boundaries of anatomical structures using an ultrasound imaging probe (2030), the method (100) comprising:
receiving (110) ultrasound imaging data (2040) for an individual (2020), wherein the ultrasound imaging data (2040) includes at least a B-mode ultrasound image (200A) of an anatomical structure of the individual (2020), the anatomical structure (1104, 1812) having tissue boundaries that define the anatomical structure (1104, 1812);
estimating (120) a rough position (302, 1102) of the anatomical structure (1104, 1812) based on at least the B-mode ultrasound image (200A) received;
determining (130) a full region-of-interest (402, 1802) for the anatomical structure (1104, 1812) based on the rough position (302, 1102) of the anatomical structure (1104, 1812);
determining (140) at least a first subsection (506, 1808) of the full region-of-interest (402, 1802) and at least a second subsection (504, 1806) of the full region-of-interest (402, 1802); and
generating (150) an enhanced ultrasound image (200C) by applying a first directional image processing technique to at least the first subsection (506, 1808) of the full region-of-interest (402, 1802) and a second directional image processing technique to at least the second subsection (504, 1806) of the full region-of-interest (402, 1802).

2. The method (100) of claim 1, wherein the rough position (302, 1102) of the anatomical structure (1104, 1812) is estimated by approximating a rough centroid of the anatomical structure (1104, 1812) or by estimating an image intensity range related to fluid within the anatomical structure (1104, 1812).

3. The method (100) of claim 2, wherein the full region-of-interest (402, 1802) is determined such that the full region-of-interest (402, 1802) is centered on the rough centroid approximated for the anatomical structure (1104, 1812).

4. The method (100) of claim 1, wherein ultrasound imaging data (2040) further comprises a Color Doppler data, and the rough position (302, 1102) of the anatomical structure (1104, 1812) is further estimated based on the Color Doppler data.

5. The method (100) of claim 1, wherein the anatomical structure (1104, 1812) is a blood vessel lumen or an organ.

6. The method (100) of claim 1, wherein the first and second directional image processing techniques are directional image filtering algorithms.

7. The method (100) of claim 6, wherein the first directional image processing technique is a first morphological filtering algorithm having a first kernel, and the second directional image processing technique is a second morphological filtering algorithm having a second kernel.

8. The method (100) of claim 7, wherein the first kernel has a different shape and/or size than the second kernel.

9. The method (100) of claim 1, further comprising:
remapping an image intensity profile of the B-mode ultrasound image (200A) to generate a processed B-mode ultrasound image (200B); and
performing a feature segmentation operation on the enhanced ultrasound image (200C) to identify the tissue boundaries of the anatomical structure (1104, 1812) of the individual (2020), wherein the enhanced B-mode ultrasound image (200C) is generated by applying at least the first and second directional image processing techniques to the processed B- mode ultrasound image (200B).

10. The method (100) of claim 9, further comprising:
approximating the anatomical structure (1104, 1812) by fitting an ellipse to a set of points representing the tissue boundaries of the anatomical structure (1104, 1812), wherein the ellipse has a major axis and a minor axis.

11. A method (1200) of monitoring blood flow of an individual, the method (1200) comprising:
detecting (1210) tissue boundaries of an anatomical structure (1104, 1812) of the individual (2020) using an ultrasound imaging probe (2030) as claimed in claim 10, wherein the anatomical structure (1104, 1812) is a blood vessel lumen;
determining (1220) probe angles *φ* and *θ* of the ultrasound imaging probe (2030),
wherein *φ* corresponds to the orientation of the major axis of the fitted ellipse with respect to a first axis (1002) on the B-mode ultrasound image, and wherein *θ* corresponds to the ratio of the minor axis to the major axis of the fitted ellipse;
measuring (1230), using the ultrasound imaging probe (2030), Doppler frequency shift data (Δ*f_{d}*) from the blood flow within the blood vessel lumen; and
determining (1240) a blood flow measurement for the individual based on the Doppler frequency shift data (Δ*f_{d}*) and the probe angles *φ* and *θ*.

12. A system (2000) for detecting tissue boundaries of anatomical structures, the system (2000) comprising:
an ultrasound imaging probe (2030) configured to generate ultrasound imaging data (2040) comprising at least B-mode ultrasound images;
a non-transitory computer-readable storage medium (2104) having stored thereon computer-readable instructions (2122); and
one or more processors (2102) in communication with the computer-readable storage medium (2104), wherein the one or more processors (2102) are configured by the computer-readable instructions (2122) stored thereon to perform the following operations: (i) receive ultrasound imaging data (2040) for an individual (2020), wherein the ultrasound imaging data (2040) includes at least a B-mode ultrasound image (200A) of an anatomical structure (1104, 1812) of the individual (2020), the anatomical structure (1104, 1812) having tissue boundaries that define the anatomical structure (1104, 1812); (ii) estimate a rough position (302, 1102) of the anatomical structure (1104, 1812) based on at least the B-mode ultrasound image (200A) received; (iii) determine a full region-of-interest (402, 1802) for the anatomical structure (1104, 1812) based on the rough position (302, 1102) of the anatomical structure (1104, 1812); (iv) determine at least a first subsection (506, 1808) of the full region-of-interest (402, 1802) and at least a second subsection (504, 1806) of the full region-of-interest (402, 1802); and (v) generate an enhanced ultrasound image (200C) by applying a first directional image processing technique to at least the first subsection (506, 1808) of the full region-of-interest (402, 1802) and a second directional image processing technique to at least the second subsection (504, 1806) of the full region-of-interest (402, 1802).

13. The system (2000) of claim 12, wherein the ultrasound imaging data (2040) for the individual (2020) is generated using the ultrasound imaging probe (2030).

14. The system (2000) of claim 12, wherein the anatomical structure (1104, 1812) is a blood vessel lumen or an organ.

15. The system (2000) of claim 12, wherein the first and second directional image processing techniques are directional image filtering algorithms.
